# EUROPEAN PATENT SPECIFICATION

(11) **EP 2 671 586 B1**
(45) Date of publication and mention of the grant of the patent: **30.05.2018**
(21) Application number: 12742008.1
(22) Date of filing: 03.02.2012
(51) Int. Cl.: A61K 31/5575, A61P 27/02, A61P 27/06, A61K 31/5513

(54) **DRUG THERAPY FOR PREVENTING OR TREATING GLAUCOMA**
MEDIKAMENTÖSE THERAPIE ZUR PRÄVENTION ODER BEHANDLUNG VON GLAUKOMEN
PHARMACOTHÉRAPIE POUR LA PRÉVENTION OU LE TRAITEMENT DU GLAUCOME

(30) Priority: 04.02.2011 JP 2011022619
(43) Date of publication of application: 11.12.2013
(73) Proprietor: KOWA CO., LTD., Naka-ku Nagoya-shi Aichi 460-8625 (JP)
(72) Inventor: MIZUNO, Ken, Higashimurayama-shi Tokyo 189-0022 (JP)
(74) Representative: Blodig, Wolfgang
(86) International application number: PCT/JP2012/052448
(87) International publication number: WO 2012/105674

(56) References cited:
- EP-A1- 1 541 151
- EP-A1- 1 818 059
- EP-A1- 1 932 841
- WO-A1-2006/137368
- WO-A1-2007/007737
- WO-A1-2007/026664
- WO-A1-2008/105058
- JP-A- 2004 107 335
- JP-A- 2006 290 827
- JP-A- 2007 084 474
- US-A1- 2010 093 789
- EISENBERG D L ET AL: "A PRELIMINARY RISK-BENEFIT ASSESSMENT OF LATANOPROST AND UNOPROSTONE IN OPEN-ANGLE GLAUCOMA AND OCULAR HYPERTENSION", DRUG SAFETY, ADIS PRESS, AUCKLAND, NZ, vol. 20, no. 6, 1 June 1999 (1999-06-01), pages 505-514, XP009014533, ISSN: 0114-5916

## Description

### [Technical Field]

The present invention relates to a drug therapy for prevention or treatment of glaucoma or ocular hypertension.

### [Background Art]

Glaucoma is a disease that the ocular pressure elevated due to various etiologies leads to damage and atrophy of the optic nerve, resulting in the abnormal visual field, and thus visual acuity is gradually reduced. Since the optic nerve does not recover once optic nerve atrophy occurs, glaucoma is a refractory disease in that not only vision is lost if glaucoma is untreated, but also the condition is only maintained even after successful treatment, and recovery cannot be expected. Furthermore, ocular hypertension, which may lead to development of glaucoma over a long time although in the absence of visual field defects, also has a similar risk.

Glaucoma is classified into three types: developmental glaucoma, secondary glaucoma, and primary glaucoma. Patients with developmental glaucoma are born with underdevelopment of angle, and obstruction of the aqueous outflow causes this type of glaucoma. Secondary glaucoma arises as a result of clear causes such as inflammation or injury and is caused by ocular co-morbidity such as uveitis or ocular injury as well as hemorrhage due to diabetes, long-term use of steroid hormones for the treatment of other diseases, and the like. Primary glaucoma is a generic name of glaucomas of types with unclear causes and occurs most commonly of glaucomas, with a high incidence among middle aged and elderly persons. Primary glaucoma and secondary glaucoma are further subdivided into two types, open-angle glaucoma and angle closure glaucoma, depending on the blockage of the aqueous outflow. While many patients develop normal tension glaucoma in the absence of elevated intraocular pressure, the primary aim of glaucoma treatment is to lower the intraocular pressure.

For the treatment of glaucoma, laser treatment (laser trabeculoplasty), surgical therapy (trabeculectomy or trabeculotomy), or the like is performed when intraocular pressure cannot be controlled with a drug or a patient with angle closure glaucoma has an acute glaucoma attack, but drug therapy is used as the first line therapy.

Drugs used in the drug therapy of glaucoma include sympathetic nerve stimulants (nonselective stimulants such as epinephrine and α2 stimulants such as apraclonidine), sympathetic nerve blockers (β blockers such as timolol, befunolol, carteolol, nipradilol, betaxolol, levobunolol, and metipranolol and α1 blockers such as bunazosin hydrochloride), parasympathetic nerve agonists (pilocarpine, etc.), carbonic anhydrase inhibitors (acetazolamide, etc.), prostaglandins (isopropyl unoprostone, latanoprost, travoprost, bimatoprost, tafluprost, etc.), and the like.

Of these drugs, a prostaglandin is a type of drug which facilitates the aqueous outflow from the uveoscleral outflow to lower intraocular pressure, and is also commonly used in clinical practice (Non-patent Literature 1).

Meanwhile, Rho kinase inhibitors have been found as candidate remedies for glaucoma based on a novel mechanism of action (Patent Literatures 1 and 2). Rho kinase inhibitors lower intraocular pressure by promoting aqueous outflow from the trabecular outflow pathway (Non Patent Literature 2), and it is further suggested that this action may be attributed to changes in the cytoskeleton of a trabecular cell (Non Patent Literatures 2 and 3).

In the treatment of glaucoma and ocular hypertension, drugs having an intraocular pressure lowering action are used in combination to enhance the intraocular pressure lowering action. For example, combination use of a prostaglandin and a sympathetic nerve blocker (Patent Literature 3), glaucoma therapy through ocular administration of a combination of some drugs having an intraocular pressure lowering action (Patent Literature 4), and the like have been reported. Furthermore, a remedy for glaucoma comprising a Rho kinase inhibitor and a prostaglandin in combination (Patent Literatures 5 to 8) have also been reported.

However, the above-mentioned known remedies and therapies for glaucoma and ocular hypertension are far from satisfactory in view of the potency of the intraocular pressure lowering effect and the duration of action. In particular, it is more difficult to lower normal intraocular pressure in patients with normal tension glaucoma than elevated intraocular pressure. The above-mentioned existing drugs and combinations thereof have limitations in the treatment of normal tension glaucoma, and enhancement of the ocular pressure lowering action is needed in the clinical setting.

### [Citation List]

### [Patent Literature]

[Patent Literature 1] WO00/09162
[Patent Literature 2] JP-A-11-349482
[Patent Literature 3] Japanese Patent No. 2726672
[Patent Literature 4] WO02/38158
[Patent Literature 5] JP-A-2004-107335
[Patent Literature 6] WO07/026664
[Patent Literature 7] WO08/105058
[Patent Literature 8] EP 1541151 A1

### [Non Patent Literature]

[Non Patent Literature 1] Journal of the Eye, 15(4), 475-480 (1998)
[Non Patent Literature 2] IOVS, 42(1), 137-144 (2001)
[Non Patent Literature 3] IOVS, 42(5), 1029-1037 (2001)

### [Summary of Invention]

### [Technical Problem]

The present invention provides a drug therapy for prevention or treatment of glaucoma or ocular hypertension with a potent intraocular pressure lowering effect and a prolonged duration of action.

### [Solution to Problem]

The inventor of the present invention conducted various researches in order to achieve the foregoing object. As a result, he has found that a potent intraocular pressure lowering effect is exerted by administering (S)-(-)-1-(4-fluoro-5-isoquinolinesulfonyl)-2-methyl-1,4-homopiperazine or a salt thereof, or a solvate thereof, and the prostaglandin latanoprost in combination, and the duration of action is prolonged.

Specifically, the present invention relates to the subject matter of claims 1 to 7, in particular to the followings.
1) A combination of (S)-(-)-1-(4-fluoro-5-isoquinolinesulfonyl)-2-methyl-1,4-homopiperazine or a salt thereof, or a solvate thereof, and a prostaglandin for prevention or treatment of glaucoma, wherein the prostaglandin is latanoprost.
2) The combination according to 1), which is prepared in one dosage form.
3) The combination according to 1), which is a kit comprising a drug comprising (S)-(-)-1-(4-fluoro-5-isoquinolinesulfonyl)-2-methyl-1,4-homopiperazine and a drug comprising latanoprost.
4) A combination of (S)-(-)-1-(4-fluoro-5-isoquinolinesulfonyl)-2-methyl-1,4-homopiperazine or a salt thereof, or a solvate thereof, and a prostaglandin for prevention or treatment of ocular hypertension, wherein the prostaglandin is latanoprost.
5) The combination according to 4), which is prepared in one dosage form.
6) The combination according to 4), which is a kit comprising a drug comprising (S)-(-)-1-(4-fluoro-5-isoquinolinesulfonyl)-2-methyl-1,4-homopiperazine and a drug comprising latanoprost.
7) A combination of (S)-(-)-1-(4-fluoro-5-isoquinolinesulfonyl)-2-methyl-1,4-homopiperazine or a salt thereof, or a solvate thereof, and a prostaglandin suitable for prevention or treatment of glaucoma or ocular hypertension, wherein the prostaglandin is latanoprost.

### [Effects of Invention]

According to the present invention, means for prevention or treatment of glaucoma or ocular hypertension with a potent intraocular pressure lowering effect and a prolonged duration of action can be provided.

### [Brief Description of Drawings]

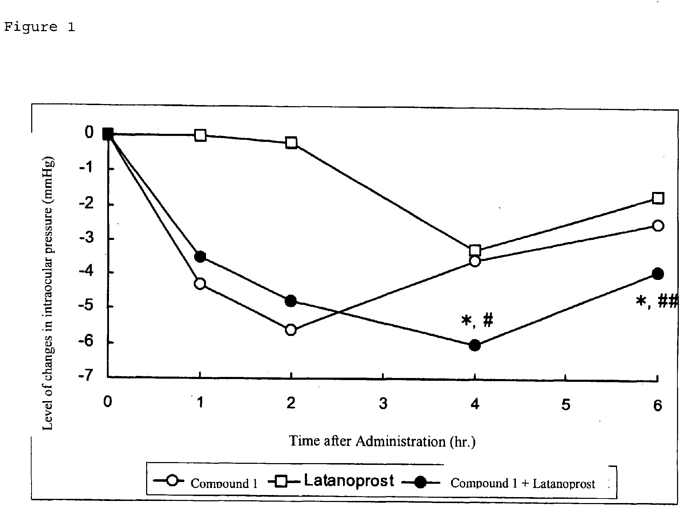

[Figure 1] Figure 1 is a graph showing changes in intraocular pressure over time in each treatment group. Intraocular pressure is shown as a change from the initial intraocular pressure (mean ± standard error). □, latanoprost (denoted as "Latanoprost") alone group; ○, (S)-(-)-1-(4-fluoro-5-isoquinolinesulfonyl)-2-methyl-1,4-homopiperazine (denoted as "Compound 1") alone group; ●, latanoprost (denoted as "Latanoprost") plus (S)-(-)-1-(4-fluoro-5-isoquinolinesulfonyl)-2-methyl-1,4-homopiperazine (denoted as "Compound 1") group; Statistical analysis: *p<0.05 vs. Compound 1 group, #p<0.05, ##p<0.01 vs. latanoprost group.

### [Embodiment for carrying out the Invention]

(S)-(-)-1-(4-fluoro-5-isoquinolinesulfonyl)-2-methyl-1,4-homopiperazine used in the present invention is a compound having antagonistic actions against substance P and leukotriene D4, and a Rho kinase inhibiting action and can be produced by known methods for example, the method described in WO99/20620.

Examples of salts of (S)-(-)-1-(4-fluoro-5-isoquinolinesulfonyl)-2-methyl-1,4-homopiperazine include salts of inorganic acids such as hydrochloric acid, sulfuric acid, nitric acid, hydrofluoric acid, and hydrobromic acid and salts of organic acids such as acetic acid, tartaric acid, lactic acid, citric acid, fumaric acid, maleic acid, succinic acid, methanesulfonic acid, ethanesulfonic acid, benzenesulfonic acid, toluenesulfonic acid, naphthalenesulfonic acid, and camphorsulfonic acid, and hydrochlorides are particularly preferred.

(S)-(-)-1-(4-fluoro-5-isoquinolinesulfonyl)-2-methyl-1,4-homopiperazine or a salt thereof can exist not only as unsolvated forms, but also as hydrates or solvates, and hydrates are preferable. However, the present invention includes all the crystalline forms of these compounds and hydrates or solvates thereof.

Meanwhile, the prostaglandin having an ocular hypotensive effect and useful in treating glaucoma used as the prostaglandin of the present invention is latanoprost disclosed in JP-A-03-501025 ((+)-Isopropyl(Z)-7-[(1R,2R,3R,5S)-3,5-dihydroxy-2-[(3R)-3-hydroxy-5-phenylpentyl]cyclopentyl]-5-heptenoate).

When (S)-(-)-1-(4-fluoro-5-isoquinolinesulfonyl)-2-methyl-1,4-homopiperazine or a salt thereof, or a solvate thereof, and the prostaglandin latanoprost are used in combination, a potent and prolonged intraocular pressure lowering effect is exhibited even from normal intraocular pressure as shown in the examples described later. Therefore, the combination is useful as a preventive or a remedy for glaucoma or ocular hypertension. In addition, such a combination may be used for manufacture of a remedy for prevention or treatment of glaucoma and/or ocular hypertension. Also, these combination preparations can be administered to a human (patient) in need of prevention or treatment of glaucoma and/or ocular hypertension, thereby preventing or treating the glaucoma and/or ocular hypertension. Here, examples of the glaucoma include primary open angle glaucoma, normal tension glaucoma, glaucoma producing excessive aqueous humor, ocular hypertension, acute angle closure glaucoma, chronic angle closure glaucoma, plateau iris syndrome, mixed glaucoma, steroid glaucoma, capsular glaucoma, pigmentary glaucoma, amyloid glaucoma, and neovascular glaucoma, malignant glaucoma, and the like. Furthermore, ocular hypertension is also called "high blood pressure of the eye" and refers to a symptom with abnormally high intraocular pressure even in the absence of any clear lesion in the optic nerve. Many high intraocular pressure conditions such as postoperative high intraocular pressure fall within the scope of the present invention.

A combination of (S)-(-)-1-(4-fluoro-5-isoquinolinesulfonyl)-2-methyl-1,4-homopiperazine or a salt thereof, or a solvate thereof, and the prostaglandin latanoprost for prevention or treatment of glaucoma or ocular hypertension of the present invention may be prepared in one dosage form comprising effective amounts of the respective drugs at a suitable mixing ratio as a combination drug or a kit used by administering each preparation comprising an effective amount of each drug simultaneously or separately at an interval.

When (S)-(-)-1-(4-fluoro-5-isoquinolinesulfonyl)-2-methyl-1,4-homopiperazine or a salt thereof, or a solvate thereof is separated from the prostaglandin latanoprost and each is individually formulated, each preparation can be prepared in accordance with a known procedure. For example, a preparation comprising (S)-(-)-1-(4-fluoro-5-isoquinolinesulfonyl)-2-methyl-1,4-homopiperazine or a salt thereof, or a solvate thereof can be prepared with reference to preparations, as an example, disclosed in the above international patent publication (WO00/09162 and WO97/23222). A preparation comprising the prostaglandin latanoprost can be prepared with reference to preparations, as an example, disclosed in Japanese Unexamined Patent Application Publication or National Publication of International Patent Application (JP-A-59-1418, JP-A-03-501025, JP-A-02-108, JP-A-08-501310, JP-A-10-182465, and JP-A-11-071344). As for commercially available existing remedies for glaucoma, in particular, such as latanoprost, isopropyl unoprostone, bimatoprost, travoprost, and tafluprost, commercially available preparations can also be used.

When a preparation comprising (S)-(-)-1-(4-fluoro-5-isoquinolinesulfonyl)-2-methyl-1,4-homopiperazine or a salt thereof, or a solvate thereof, and the prostaglandin latanoprost is prepared, the preparation can also be prepared in accordance with a known procedure. For example, a tonicity agent, a buffer, a surfactant, a preservative, and/or other agents can be used depending on the need to prepare eye drops. A pH may be within a range permissible for an ophthalmic preparation, and is preferably a range from pH 4 to 8.

The above-mentioned preparations may be preferably used as an ophthalmological preparation, particularly preferably as an instillation, and such an ophthalmic preparation may be aqueous eye drop, nonaqueous eye drop, suspension eye drop, emulsion eye drop, eye ointment, and the like. Such preparation can be produced by (preparation) methods known to those skilled in the art as a composition suitable for the administration route by adding pharmacologically acceptable carriers such as, for example, tonicity agents, chelating agents, stabilizers, pH modifiers, preservatives, antioxidants, solubilizing agents, and thickening agents, if necessary.

An ophthalmic preparation can be prepared by, for example, dissolving or suspending desired ingredients of the above-mentioned compounds in an aqueous solvent such as sterilized purified water or physiological saline or a nonaqueous solvent such as vegetable oil such as cottonseed oil, soybean oil, sesame oil, or peanut oil at a predetermined osmotic pressure and subjecting the solution or suspension to sterilization treatment such as sterilization by filtration. When an eye ointment is prepared, an ointment vehicle can be added in addition to the above-mentioned various ingredients. The above-mentioned ointment vehicle is not particularly limited, but preferred examples thereof include oily vehicles such as vaseline, liquid paraffin, and polyethylene, emulsion vehicles obtained by emulsifying the oil phase and the aqueous phase with a surfactant or the like, watersoluble vehicles comprising hydroxypropylmethylcellulose, carboxymethylcellulose, polyethylene glycol or the like, and so forth.

When a kit for prevention or treatment of glaucoma or ocular hypertension includes a combination of (S)-(-)-1-(4-fluoro-5-isoquinolinesulfonyl)-2-methyl-1,4-homopiperazine or a salt thereof, or a solvate thereof, and the prostaglandin latanoprost, each drug is packed into a separate package, each drug comprising either the prostaglandin or (S)-(-)-1-(4-fluoro-5-isoquinolinesulfonyl)-2-methyl-1,4-homopiperazine or a salt thereof, or a solvate thereof as so formulated. Each pharmaceutical agent can be taken out from each package at the time of administration, and can be used in this regimen. Furthermore, both the drug preparations can be packaged in a form suitable for combination use for each dose.

When (S)-(-)-1-(4-fluoro-5-isoquinolinesulfonyl)-2-methyl-1,4-homopiperazine or a salt thereof, or a solvate thereof, and the prostaglandin latanoprost are combined to be used for prevention or treatment of glaucoma or ocular hypertension, the dose varies depending on the patient's body weight, age, sex, symptom, dose form, number of dosages, and the like, but the usual adult daily dose of (S)-(-)-1-(4-fluoro-5-isoquinolinesulfonyl)-2-methyl-1,4-homopiperazine or a salt thereof, or a solvate thereof is in the range of 0.025 to 10000 µg, preferably 0.025 to 2000 µg, and more preferably 0.1 to 2000 µg. The usual adult daily dose of the prostaglandin is in the range of 0.1 to 1000 µg and preferably 1 to 300 µg. Specific examples of the prostaglandin dose include a usual daily dose of 1 to 5 µg of latanoprost.

When a preparation comprising (S)-(-)-1-(4-fluoro-5-isoquinolinesulfonyl)-2-methyl-1,4-homopiperazine or a salt thereof, or a solvate thereof, and the prostaglandin latanoprost is to be administered, a blending ratio is appropriately selected to prepare the preparation so that each daily dose is within the above amount or less of each component.

The number of dosages is not particularly limited, but it is preferable to administer the dose once daily or divide the dose into several times. One to several drops of a liquid eye drop can be instilled as one dose. When the preparations are packaged as a kit, the individual preparations may be administered simultaneously or at an interval of 5 minutes to 24 hours.

The present invention will be explained more specifically with reference to the following examples. However, the scope of the present invention is not limited to these examples.

### [Examples]

### Example 1

To examine usefulness of combination use of (S)-(-)-1-(4-fluoro-5-isoquinolinesulfonyl)-2-methyl-1,4-homopiperazine and a prostaglandin, the intraocular pressure lowering effects were compared by administering either of these drugs alone or both in combination to laboratory animals.

### 1. Preparation of test compound solutions

### A. Preparation of (S)-(-)-1-(4-fluoro-5-isoquinolinesulfonyl)-2-methyl-1,4-homopiperazine solution

(S)-(-)-1-(4-fluoro-5-isoquinolinesulfonyl)-2-methyl-1,4-homopiperazine-hydrochloride dihydrate was dissolved in physiological saline, and the solution was neutralized (pH 6.0) by adding sodium dihydrogenphosphate and sodium hydroxide to prepare a (S)-(-)-1-(4-fluoro-5-isoquinolinesulfonyl)-2-methyl-1,4-homopiperazine solution at a desired concentration.

### B. Preparation of prostaglandin

The commercially available Latanoprost (product of Pfizer Inc., 0.005% ophthalmic solution) was used as it was.

### 2. Test method

The intraocular pressure lowering effect was examined after the combination use of (S)-(-)-1-(4-fluoro-5-isoquinolinesulfonyl)-2-methyl-1,4-homopiperazine and latanoprost. As a control, the intraocular pressure lowering effect was also examined after the use of (S)-(-)-1-(4-fluoro-5-isoquinolinesulfonyl)-2-methyl-1,4-homopiperazine or latanoprost alone.

### A. Drugs and animals used in test

(S)-(-)-1-(4-fluoro-5-isoquinolinesulfonyl)-2-methyl-1,4-homopiperazine solution: 0.4% solution (instillation volume, 20 µL)
Latanoprost: 0.005% ophthalmic solution (instillation volume, 20 µL)
Laboratory animals: Macaca fascicularis (sex: male, 5 animals per group)

### B. Administration and measurement

### (1) Administration of two drugs in combination

1) One drop of 0.4% oxybuprocaine hydrochloride ophthalmic solution (trade name: Benoxil ophthalmic Solution 0.4%) was instilled in both eyes of the laboratory animals for local anesthesia (only data for the instilled eyes are shown).
2) Intraocular pressure was measured immediately before administration of the test compound solution as the initial intraocular pressure.
3) The (S)-(-)-1-(4-fluoro-5-isoquinolinesulfonyl)-2-methyl-1,4-homopiperazine solution was instilled in one eye of the laboratory animals, followed by the instillation of latanoprost solution in the same eye.
4) At 1 hour, 2 hours, 4 hours, and 6 hours after the instillation of both drugs, one drop each of 0.4% oxybuprocaine hydrochloride ophthalmic solution was instilled in both eyes for local anesthesia, and intraocular pressure was measured.

### (2) Administration of (S)-(-)-1-(4-fluoro-5-isoquinolinesulfonyl)-2-methyl-1,4-homopiperazine alone

The (S)-(-)-1-(4-fluoro-5-isoquinolinesulfonyl)-2-methyl-1,4-homopiperazine alone was instilled, and then tests were performed at the same measurement timings as in the above-described combination use test.

### (3) Administration of Latanoprost Alone

Latanoprost was used as a substitute for the above test solution used in administration of the above (S)-(-)-1-(4-fluoro-5-isoquinolinesulfonyl)-2-methyl-1,4-homopiperazine alone. Except for that, the same method was used to perform the above sole administration test.

### 3. Results and discussion

The test results are shown in Figure 1. Intraocular pressures are shown as changes from the initial intraocular pressure. Microsoft Excel 2000 SP-3, Paired t-test was used for statistical processing.

As shown in Figure 1, a superior intraocular pressure lowering effect was observed in the (S)-(-)-1-(4-fluoro-5-isoquinolinesulfonyl)-2-methyl-1,4-homopiperazine plus latanoprost group to those in the single drug treatment groups, i.e., the (S)-(-)-1-(4-fluoro-5-isoquinolinesulfonyl)-2-methyl-1,4-homopiperazine alone group and the latanoprost alone group, showing improvement of the prolonged action.

The above results revealed that a potent intraocular pressure lowering effect and improvement of the prolonged action could be obtained by using latanoprost and (S)-(-)-1-(4-fluoro-5-isoquinolinesulfonyl)-2-methyl-1,4-homopiperazine in combination.

### [Industrial Applicability]

A combination of (S)-(-)-1-(4-fluoro-5-isoquinolinesulfonyl)-2-methyl-1,4-homopiperazine or a salt thereof, or a solvate thereof, and the prostaglandin latanoprost according to the present invention has an excellent ocular hypotensive effect and prolonged effect thereof, and is therefore useful as a remedy for prevention or treatment of glaucoma or ocular hypertension.

## Claims

1. A combination of (S)-(-)-1-(4-fluoro-5-isoquinolinesulfonyl)-2-methyl-1,4-homopiperazine or a salt thereof, or a solvate thereof, and a prostaglandin for use in a method for prevention or treatment of glaucoma, wherein the prostaglandin is latanoprost.

2. The combination for the use according to claim 1, which is prepared in one dosage form.

3. The combination for the use according to claim 1, which is a kit comprising a drug comprising (S)-(-)-1-(4-fluoro-5-isoquinolinesulfonyl)-2-methyl-1,4-homopiperazine and a drug comprising latanoprost.

4. A combination of (S)-(-)-1-(4-fluoro-5-isoquinolinesulfonyl)-2-methyl-1,4-homopiperazine or a salt thereof, or a solvate thereof, and a prostaglandin for use in a method for prevention or treatment of ocular hypertension, wherein the prostaglandin is latanoprost.

5. The combination for the use according to claim 4, which is prepared in one dosage form.

6. The combination for the use according to claim 4, which is a kit comprising a drug comprising (S)-(-)-1-(4-fluoro-5-isoquinolinesulfonyl)-2-methyl-1,4-homopiperazine and a drug comprising latanoprost.

7. A combination of (S)-(-)-1-(4-fluoro-5-isoquinolinesulfonyl)-2-methyl-1,4-homopiperazine or a salt thereof, or a solvate thereof, and a prostaglandin suitable for prevention or treatment of glaucoma or ocular hypertension, wherein the prostaglandin is latanoprost.

## Patentansprüche

1. Kombination aus (S)-(-)-1-(4-Fluor-5-isochinolinsulfonyl)-2-methyl-1,4-homopiperazin oder eines Salzes desselben oder eines Solvats desselben und einem Prostaglandin, zur Verwendung in einem Verfahren zur Behandlung eines Glaukoms, wobei das Prostaglandin Latanoprost ist.

2. Kombination zur Verwendung nach Anspruch 1, welche als eine Dosierungsform hergestellt wird.

3. Kombination zur Verwendung nach Anspruch 1, welche ein Kit ist, umfassend ein Arzneimittel, das (S)-(-)-1-(4-Fluor-5-isochinolinsulfonyl)-2-methyl-1,4-homopiperazin umfasst, und ein Arzneimittel, das Latanoprost umfasst.

4. Kombination aus (S)-(-)-1-(4-Fluor-5-isochinolinsulfonyl)-2-methyl-1,4-homopiperazin oder eines Salzes desselben oder eines Solvats desselben und einem Prostaglandin, zur Verwendung in einem Verfahren zur Prävention oder zur Behandlung von okulärer Hypertension, wobei das Prostaglandin Latanoprost ist.

5. Kombination zur Verwendung nach Anspruch 4, welche als eine Dosierungsform hergestellt wird.

6. Kombination zur Verwendung nach Anspruch 4, welche ein Kit ist, umfassend ein Arzneimittel, das (S)-(-)-1-(4-Fluor-5-isochinolinsulfonyl)-2-methyl-1,4-homopiperazin umfasst, und ein Arzneimittel, das Latanoprost umfasst.

7. Kombination aus (S)-(-)-1-(4-Fluor-5-isochinolinsulfonyl)-2-methyl-1,4-homopiperazin oder eines Salzes desselben oder eines Solvats desselben und einem Prostaglandin, das zur Prävention oder zur Behandlung eines Glaukoms oder okulärer Hypertension geeignet ist, wobei das Prostaglandin Latanoprost ist.

## Revendications

1. Combinaison de (S)-(-)-1-(4-fluoro-5-isoquinolinesulfonyl)-2-méthyl-1,4-homopipérazine ou d'un sel de celle-ci, ou d'un solvate de celle-ci, et d'une prostaglandine, pour une utilisation dans une méthode de prévention ou de traitement du glaucome, dans laquelle la prostaglandine est le latanoprost.

2. Combinaison pour une utilisation selon la revendication 1, qui est préparée sous une forme posologique unitaire.

3. Combinaison pour une utilisation selon la revendication 1, qui est un kit comprenant un médicament comprenant de la (S)-(-)-1-(4-fluoro-5-isoquinolinesulfonyl)-2-méthyl-1,4-homopipérazine et un médicament comprenant du latanoprost.

4. Combinaison de (S)-(-)-1-(4-fluoro-5-isoquinolinesulfonyl)-2-méthyl-1,4-homopipérazine ou d'un sel de celle-ci, ou d'un solvate de celle-ci, et d'une prostaglandine, pour une utilisation dans une méthode de prévention ou de traitement de l'hypertension oculaire, dans laquelle la prostaglandine est le latanoprost.

5. Combinaison pour une utilisation selon la revendication 4, qui est préparée sous une forme posologique unitaire.

6. Combinaison pour une utilisation selon la revendication 4, qui est un kit comprenant un médicament comprenant de la (S)-(-)-1-(4-fluoro-5-isoquinolinesulfonyl)-2-méthyl-1,4-homopipérazine et un médicament comprenant du latanoprost.

7. Combinaison de (S)-(-)-1-(4-fluoro-5-isoquinolinesulfonyl)-2-méthyl-1,4-homopipérazine ou d'un sel de celle-ci, ou d'un solvate de celle-ci, et d'une prostaglandine convenant pour la prévention ou le traitement du glaucome ou de l'hypertension oculaire, dans laquelle la prostaglandine est le latanoprost.
